# EUROPÄISCHE PATENTANMELDUNG

(11) **EP 1 932 890 A2**
(43) Veröffentlichungstag der Anmeldung: **18.06.2008**
(21) Anmeldenummer: 07019976.5
(22) Anmeldetag: 12.10.2007
(51) Int. Cl.: C09D 5/02, C09D 5/14

(54) **Dispersion enthaltend nanoskalige Silber enthaltende Partikel und Verfahren zur Behandlung von Oberflächen**

(30) Priorität: 11.12.2006 DE 102006058596
(71) Anmelder: Carl Freudenberg KG, 69469 Weinheim (DE)
(72) Erfinder: Rühle, Thomas, Dr., 68259 Mannheim (DE); Schubert, Dirk W., Dr., 69493 Hirschberg (DE); Henke, Jürgen, 68519 Viernheim (DE); Gruber, Achim, 69250 Schönau (DE); Stangler, Stefan, Dr., 69514 Laudenbach (DE)

(57) **Zusammenfassung**

Eine Dispersion, enthaltend zumindest eine Flüssigkeit, in welcher Silber enthaltende Partikel dispergiert vorliegen, wobei die Silber enthaltenden Partikel nanoskalig dimensioniert sind sowie ein Verfahren zur Behandlung von Oberflächen, wobei eine solche Dispersion auf eine Oberfläche aufgebracht wird, lösen die Aufgabe, Oberflächen derart zu behandeln, dass diese mit schneller und zugleich lang anhaltender Wirkung desinfiziert und/oder antimikrobiell derart ausgerüstet werden, dass die Ausbildung von Gerüchen wirksam gehemmt wird.

## Beschreibung

### Technisches Gebiet

Die Erfindung betrifft eine Dispersion und ein Verfahren zur Behandlung von Oberflächen, wobei eine Dispersion auf eine Oberfläche aufgebracht wird.

### Stand der Technik

Dispersionen und Verfahren zur Behandlung von Oberflächen, bei denen Dispersionen als Beschichtungsmassen verwendet werden, sind aus dem Stand der Technik bereits bekannt. Die bekannten Dispersionen sind jedoch nur wenig geeignet, Oberflächen zu desinfizieren oder antimikrobiell derart auszurüsten, dass die Entstehung von Gerüchen gehemmt wird. Eine antimikrobiell ausgerüstete Oberfläche kann Keimbildungen unterdrücken und Bakterien, Pilze oder Viren abtöten.

### Darstellung der Erfindung

Der Erfindung liegt daher die Aufgabe zugrunde, Oberflächen derart zu behandeln, dass diese mit schneller und zugleich lang anhaltender Wirkung desinfiziert und/oder antimikrobiell derart ausgerüstet werden, dass die Ausbildung von Gerüchen wirksam gehemmt wird.

Die vorliegende Erfindung löst die zuvor genannte Aufgabe durch die Merkmale des Patentanspruchs 1. Danach enthält eine Dispersion zumindest eine Flüssigkeit, in welcher Silber enthaltende Partikel dispergiert vorliegen, wobei die Partikel nanoskalig dimensioniert sind.

Erfindungsgemäß ist erkannt worden, dass eine Dispersion, in welcher nanoskalige, Silber enthaltende Partikel vorliegen, Oberflächen sehr schnell desinfizieren kann. Unter nanoskaligen Partikeln werden im Sinne dieser Anmeldung Partikel verstanden, welche in zumindest einer Dimension eine Ausdehnung aufweisen, die sich im Bereich < 1000 nm bewegt. Dabei ist durchaus denkbar, dass ein Partikel zwar eine Breite aufweist, die geringer als 1000 nm ist, jedoch eine Länge, die größer als 1000 nm ist. Die rasche Desinfizierungswirkung hängt mit der schnellen Mobilisierbarkeit der antimikrobiell wirkenden Ag+ - Ionen (Silberionen) zusammen.

Erfindungsgemäß ist insbesondere erkannt worden, dass eine Oberfläche, auf welcher eine erfindungsgemäße Dispersion aufgebracht wird, für einen langen Zeitraum desinfizierbar ist. Aufgrund einer sehr langen Verweildauer der nanoskaligen Partikel auf der Oberfläche ist eine lang anhaltende Desinfizierung der Oberfläche gewährleistet. Ganz konkret ist erkannt worden, dass sich die nanoskaligen Partikel aufgrund ihrer geringen Ausdehnung in sehr feine Zerklüftungen von Oberflächen setzen und dort verweilen können. Insoweit ist es möglich, eine Oberfläche mit schneller und zugleich lang anhaltender Wirkung derart zu desinfizieren, dass die Ausbildung von Gerüchen wirksam gehemmt wird.

Folglich ist die genannte Aufgabe gelöst.

Die Silber enthaltenden Partikel könnten mittlere Durchmesser aufweisen, die höchstens 500 nm betragen. Dabei wird angenommen, dass die Partikel im Wesentlichen kugelförmig ausgestaltet sind. Sofern ein Partikel von der Kugelform abweicht, wird zur Ermittlung eines mittleren Durchmessers dessen Masse einer Kugel zugeordnet. Diese fiktive Kugel weist dann einen fiktiven mittleren Durchmesser auf. Ein mittlerer Durchmesser, der geringer als 500 nm ist, erlaubt die Realisierung einer Dispersion mit sehr hoher Silberoberfläche.

Bevorzugt könnten die Partikel einen mittleren Durchmesser von 5 bis 500 nm aufweisen. Die Auswahl des mittleren Durchmessers aus diesem Zahlenbereich bewirkt auf überraschende Weise, dass die Dispersion einerseits eine hohe Silberoberfläche zur Verfügung stellt und andererseits eine Viskosität aufweist, die ein problemloses Aufbringen der Dispersion auf Oberflächen erlaubt.

Besonders bevorzugt könnte der mittlere Durchmesser 50 bis 500 nm betragen. Die Auswahl des mittleren Durchmessers aus diesem Bereich erlaubt die Realisierung einer sehr flüssigen Dispersion, die auf eine Oberfläche aufsprühbar ist. Der Verzicht auf Partikel, die kleiner als 50 nm sind, bewirkt eine Erniedrigung der Viskosität der Dispersion.

Die Dispersion könnte als Emulsion oder als Suspension ausgestaltet sein. Emulsionen und Suspensionen lassen sich gleichmäßig verteilt und mit sehr geringer Schichtdicke auf Oberflächen aufbringen, da sie eine sprühfähige oder streichfähige Konsistenz aufweisen können.

Die Flüssigkeiten könnten wässrige und/ oder alkoholische Lösungen umfassen. Dabei ist ganz konkret denkbar, dass alkoholische Lösung mit wässrigen gemischt vorliegen, wobei in der Mischung Partikel dispergiert sind. Die Verwendung von alkoholischen Lösungen unterstützt die desinfizierende Wirkung der Beschichtungsmasse. Die Verwendung wässriger Lösungen, insbesondere die Verwendung von Wasser, erlaubt eine kostengünstige Herstellung der Dispersion, da auf Einrichtungen für den Explosionsschutz (EX - Schutz) verzichtet werden kann.

Die Dispersion könnte ein Tensid enthalten. Vor diesem Hintergrund ist denkbar, dass der Dispersion ein Tensid beigemengt ist, um eine gleichmäßige Verteilung der Dispersion auf einer Oberfläche zu erzielen. Des Weiteren kann die Zugabe eines Tensids bewirken, dass eine Oberfläche problemlos benetzbar ist. Schließlich könnte der Dispersion ein Tensid beigemengt sein, um die Dispersion zu stabilisieren. Ganz konkret könnte dabei vermieden werden, dass sich einzelne Partikel in der Dispersion bei längerer Lagerung absetzen oder agglomerieren.

Als Tenside könnten ionische oder nicht-ionische Tenside verwendet werden. Ganz konkret ist denkbar, Fettalkohol-Polyglykolether, Alkylsulphonate und deren Salze zu verwenden. Die Verwendung dieser Tenside hat sich als besonders vorteilhaft erwiesen, um die Benetzung einer zu behandelnden Oberfläche zu optimieren.

Als besonders vorteilhaftes Tensid kann sich das Fettalkoholethoxylat Lutensol AO 11 der BASF AG erweisen. Die Verwendung dieses Tensids erlaubt eine starke Verdünnung von Silberdispersionen, ohne dass die Silberpartikel ausfallen oder agglomerieren.

Der Dispersion könnte ein Bindemittel beigemengt sein. Durch diese konkrete Ausgestaltung wird vorteilhaft realisiert, dass die Partikel auf einer zu behandelnden Oberfläche anhaften. Als besonders vorteilhafte Bindemittel könnten sich Acronal S 888S der BTC, BASF AG sowie iSys MTX der CHT GmbH, Tübingen erweisen.

Die Dispersion könnte einen Anteil an Wasser von 68 bis 100 Gewichtsprozent und einen Anteil an Tensid von 0 bis 7 Gewichtsprozent aufweisen. Eine Dispersion dieser Zusammensetzung ist besonders stabil, wobei die Silber enthaltenden Partikel nicht ausfallen oder agglomerieren.

Vor diesem Hintergrund könnte die Dispersion einen Anteil an Bindemittel von 5 bis 20 Gewichtsprozent aufweisen. Dieser Anteil an Bindemittel erlaubt die Erzeugung einer stabilen Dispersion, deren Silber enthaltende Partikel gut auf einer zu beschichtenden Oberfläche haften.

Der Dispersion könnten weitere Additive beigemengt sein. Die Additive könnten die Verteilung der Partikel innerhalb der Dispersion oder auf einer zu behandelnden Oberfläche begünstigen. Des Weiteren könnten die Additive zur Einstellung der Viskosität der Dispersion verwendet werden.

Der Dispersion könnten Polymere beigemengt sein. Vor diesem Hintergrund ist ganz konkret denkbar, dass die Dispersion, welche Partikel umfasst, mit einer Latex- oder Polymerdispersion gemischt wird. Eine solche Mischung bildet auf einer zu behandelnden Oberfläche Filme aus, wodurch ein zu schnelles Auswaschen der Silber enthaltenden Partikel vermieden wird. Des Weiteren kann die Filmbildung bewirken, dass die Abgabe der Partikel von der Oberfläche zeitlich gesteuert wird. Die Verwendung von Polymeren erlaubt insbesondere die Einstellung der rheologischen Eigenschaften der Dispersion. Hierbei ist konkret denkbar, dass die Dispersion derart modifiziert ist, dass sie auf eine Oberfläche weitgehend tropffrei aufgetragen werden kann.

Die genannte Aufgabe wird des Weiteren mit einem Verfahren zur Behandlung von Oberflächen gelöst, bei welchem die erfindungsgemäße Dispersion auf eine Oberfläche als Beschichtungsmasse aufgebracht wird.

Die Dispersion könnte durch Sprühköpfe auf die Oberfläche aufgesprüht werden. Die Verwendung von Sprühköpfen erlaubt einen sehr schnellen und gleichmäßigen Auftrag der Dispersion auf die Oberfläche, wenn die Dispersion sehr flüssig und niedrigviskos vorliegt.

Vor diesem Hintergrund ist auch denkbar, dass die Dispersion auf die Oberfläche aufgestrichen, aufgerollt, aufgerakelt oder aufgespachtelt wird. Diese Verfahrensschritte erlauben das Auftragen einer besonders dicken Schicht der Dispersion und erfordern eine hohe Viskosität derselben. Eine dicke Schicht kann besonders viele Silber enthaltende Partikel aufnehmen und somit einen sehr lang anhaltenden Desinfizierungseffekt auf der Oberfläche bewirken.

Denkbar ist auch, eine Oberfläche in die Dispersion einzutauchen.

Die Oberfläche könnte bei einer Temperatur getrocknet werden, die oberhalb des Siedepunkts der Flüssigkeit oder der Flüssigkeiten liegt. Dieser Verfahrensschritt erlaubt ein vollständiges Verdampfen der Flüssigkeit, so dass die Partikel isoliert auf der Oberfläche vorliegen und somit ihre antimikrobielle und desinfizierende Wirkung entfalten können.

Die Oberfläche könnte bei einer Temperatur getrocknet werden, die unterhalb des Siedepunkts der Flüssigkeit oder der Flüssigkeiten liegt. Durch Anwendung dieses Verfahrensschrittes ist vorteilhaft realisiert, dass die Partikel über einen langen Zeitraum mobil bleiben, da auf der Oberfläche über lange Zeit ein Flüssigkeitsfilm'verbleibt, in dem sich die Partikel bewegen können.

Bei den Trocknungsprozessen könnten Öfen, Heizwalzen, Strahler oder Gebläse verwendet werden. Die Verwendung dieser Einrichtungen beschleunigt den Trocknungsprozess.

Die Desinfizierung einer Oberfläche und deren antimikrobielle Ausrüstung bewirkt vorteilhaft eine Geruchshemmung und die Unterdrückung einer Keimbildung. Dieser Effekt eignet die Dispersion als Beschichtungsmasse für Zuluft- oder Abluftanlagen. Insbesondere in Zuluft- oder Abluftanlagen von Krankenhäusern ist diese Verwendung besonders vorteilhaft.

Die Beschichtung Abluft führender Rohrleitungen erlaubt die wirksame Unterdrückung unangenehmer Gerüche. Vor diesem Hintergrund ist denkbar, die Dispersion in landwirtschaftlichen Betrieben zur Beschichtung von Rohrleitungen zu verwenden. Durch die Führung von Fäkalgasen durch Abluftleitungen entstehen üblicherweise für die Umgebung unangenehme Gerüche. Diese können mit der hier beschriebenen Dispersion wesentlich vermindert werden.

Denkbar ist auch, die Dispersion in Kläranlagen oder Kompostieranlagen zu verwenden, um unangenehme Gerüche zu verhindern.

Die Dispersion könnte verwendet werden, um Schläuche und Rohre aus Polymeren zu beschichten. Diese konkrete Anwendung erlaubt einen Schutz der Polymere gegen Pilzbefall und Fouling, insbesondere wenn die Schläuche im Erdreich verlegt sind.

Die Dispersion könnte auf textile Stoffe aufgetragen werden. Konkret ist denkbar, dass die Stoffe mit der Dispersion imprägniert werden. Die Ausrüstung der Stoffe mit Dispersion bewirkt eine antimikrobielle Ausrüstung der Stoffe. Neben Kleidungsstücken könnten auch Verbandsmaterialien mit der Dispersion versehen werden, um die Wundheilung zu unterstützen und Wundinfektionen zu vermeiden.

Das hier beschriebene Verfahren zur Behandlung von Oberflächen könnte mit weiteren Verfahrensschritten kombiniert werden, die dem Korrosionsschutz dienen. Hierdurch ist vorteilhaft gewährleistet, dass eine Oberfläche einerseits gegen Korrosion und andererseits gegen die Ausbildung von Bakterien und Keimen geschützt wird.

Zur Herstellung der Dispersion benötigt man ein Lösungsmittel, idealerweise Wasser, und eine antimikrobiell wirksame Substanz. Die Substanz kann ein Reinstoff organischer oder anorganischer Natur sein. Die Substanz kann auch als Zubereitung vorliegen. Besonders geeignet sind Silber und seine Verbindungen in feinster Verteilung.

Gegebenenfalls werden der Dispersion Hilfsmittel zum Erreichen einer stabilen Suspension oder Emulsion beigemengt. Als Hilfsmittel können oberflächenaktive Stoffe und Tenside fungieren. Weiterhin dienen diese Hilfsmittel der Sicherung von ausreichenden Benetzungseigenschaften auf der zu beschichtenden Oberfläche.

Der Dispersion können Bindemittel zur Fixierung der antimikrobiell wirksamen Substanz auf der Oberfläche beigemengt werden. Weitere Hilfsmittel, die der Dispersion beigemengt werden, können Farbstoffe oder Viskositätsregler sein

Die Dispersion kann durch Mischen, Rühren, Dispergieren, Suspendieren oder Emulgieren hergestellt werden. Feinste Partikel werden teilweise auch in Situ generiert und homogen verteilt.

Nanoskalige Silberpartikel könnten durch elektrochemische Prozesse hergestellt werden.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche, andererseits auf die nachfolgende Erläuterung bevorzugter Ausführungsbeispiele der erfindungsgemäßen Lehre zu verweisen.

In Verbindung mit der Erläuterung der bevorzugten Ausführungsbeispiele werden auch im Allgemeinen bevorzugte Ausgestaltungen und Weiterbildungen der Lehre erläutert.

### Kurzbeschreibung der Tabellen

Tabelle 1 zeigt die Bestandteile der Dispersionen der Ausführungsbeispiele nach Reihe 1 in Gewichtsprozent.

Tabelle 2 zeigt die Bestandteile der Dispersionen der Ausführungsbeispiele nach Reihe 2 in Gewichtsprozent.

### Ausführung der Erfindung

Ausführungsbeispiele nach Reihe 1:

In vorgelegtem vollentsalztem Wasser (VE-Wasser) wird als Tensid das Fettalkoholethoxylat Lutensol AO 11 (BASF AG) in den in Tabelle 1 angegebenen Mengen gelöst und unter Rühren die Silberdispersion Ag - Pure (rent a scientist GmbH) zugegeben.

Es wird noch 15 Minuten zur Homogenisierung nachgerührt.

Die entstehenden verdünnten Silberdispersionen sind derart stabil, dass keine Silberpartikel ausfallen oder agglomerieren.

Mit diesen Dispersionen wurden Glas- und Metallblechproben durch Tauchen beschichtet. Nach dem Abtropfen wurden die Proben in einem Umluftofen bei 120° C getrocknet. Auf den Proben verbleibt ein leichter Schleier an mikrobiologisch aktiver Substanz, nämlich metallisches nanoskaliges Silber mit einer nanoskaligen Oxidschicht.

Ausführungsbeispiele nach Reihe 2:

In vorgelegtem VE-Wasser wird das Tensid Lutensol AO 11 (BASF AG) in den in Tabelle 2 angegebenen Mengen unter Rühren gelöst. Das Bindemittel iSys MTX (CHT GmbH) wird durch einen Dissolver emulgiert und unter Rühren wird die Silberdispersion iSys AG (CHT GmbH) zugegeben.

Es wird noch 15 Minuten zur Homogenisierung nachgerührt.

Die entstehenden verdünnten Silberdispersionen sind derart stabil, dass keine Silberpartikel ausfallen oder agglomerieren.

Mit diesen Dispersionen wurden Vliesstoffproben mit einem in der Textilindustrie üblichen Foulard durch Tauchen und Abquetschen imprägniert. Die Trocknung erfolgte in einem Umluftofen bei 120°C über 15 Minuten. Die Fixierung des Bindemittels auf den Fasern des Vliesstoffes erfolgt bei 160°C über 5 Minuten. Es entsteht ein waschbeständiger, antimikrobiell wirkender Vliesstoff.

Hinsichtlich weiterer vorteilhafter Ausgestaltungen und Weiterbildungen der erfindungsgemäßen Lehre wird einerseits auf den allgemeinen Teil der Beschreibung und andererseits auf die beigefügten Patentansprüche verwiesen.

Abschließend sei ganz besonders hervorgehoben, dass die zuvor rein willkürlich ausgewählten Ausführungsbeispiele lediglich zur Erörterung der erfindungsgemäßen Lehre dienen, diese jedoch nicht auf diese Ausführungsbeispiele einschränken.

### Tabellen

**Tabelle 1: Bestandteile der Dispersionen der Ausführungsbeispiele nach Reihe 1 in Gewichtsprozent.**

| | |
|---|---|
| VE-Wasser | 88-100 |
| Tensid | 0-7 |
| Silber-Dispersion | 0-5 |

**Tabelle 2: Bestandteile der Dispersionen der Ausführungsbeispiele nach Reihe 2 in Gewichtsprozent.**

| | |
|---|---|
| VE-Wasser | 68 - 95 |
| Tensid | 0 - 7 |
| Bindemittel | 5 - 20 |
| Silber-Dispersion | 0 - 5 |

## Patentansprüche

1. Dispersion, enthaltend zumindest eine Flüssigkeit, in welcher Silber enthaltende Partikel dispergiert vorliegen, wobei die Partikel nanoskalig dimensioniert sind.

2. Dispersion nach Anspruch 1, **dadurch gekennzeichnet, dass** die Partikel mittlere Durchmesser aufweisen, die höchstens 500 nm, bevorzugt 5 bis 500 nm, besonders bevorzugt, 50 bis 500 nm betragen.

3. Dispersion nach Anspruch 1 oder 2, **gekennzeichnet durch** eine Ausgestaltung als Emulsion.

4. Dispersion nach Anspruch 1 oder 2, **gekennzeichnet durch** eine Ausgestaltung als Suspension.

5. Dispersion nach einem der Ansprüche 1 bis 4, **dadurch gekennzeichnet, dass** die Flüssigkeiten wässrige und/ oder alkoholische Lösungen umfassen.

6. Dispersion nach einem der Ansprüche 1 bis 5, enthaltend ein Tensid.

7. Dispersion nach einem der Ansprüche 1 bis 6, enthaltend ein Bindemittel.

8. Dispersion nach einem der Ansprüche 1 bis 7, enthaltend Polymere.

9. Dispersion nach einem der Ansprüche 1 bis 8, **gekennzeichnet durch** einen Anteil an Wasser von 68 bis 100 Gewichtsprozent und einen Anteil an Tensid von 0 bis 7 Gewichtsprozent.

10. Dispersion nach Anspruch 9, **gekennzeichnet durch** einen Anteil an Bindemittel von 5 bis 20 Gewichtsprozent.

11. Verfahren zur Behandlung von Oberflächen, wobei eine Dispersion nach einem der voranstehenden Ansprüche auf eine Oberfläche aufgebracht wird.

12. Verfahren nach Anspruch 11, **dadurch gekennzeichnet, dass** die Dispersion durch Sprühköpfe auf die Oberfläche aufgesprüht wird.

13. Verfahren nach Anspruch 11 oder 12, **dadurch gekennzeichnet, dass** die Oberfläche bei einer Temperatur getrocknet wird, die oberhalb des Siedepunkts der Flüssigkeit liegt.

14. Verfahren nach Anspruch 11 oder 12, **dadurch gekennzeichnet, dass** die Oberfläche bei einer Temperatur getrocknet wird, die unterhalb des Siedepunkts der Flüssigkeit liegt.

15. Verfahren nach einem der Ansprüche 11 bis 14, **dadurch gekennzeichnet, dass** die Dispersion auf Oberflächen in Zuluft- oder Abluftanlagen aufgebracht wird.

16. Verfahren nach einem der Ansprüche 11 bis 14, **dadurch gekennzeichnet, dass** die Dispersion auf Oberflächen von Schläuchen oder Rohren aufgebracht wird, die aus Polymeren bestehen.
